## Europäisches Patentamt
## European Patent Office
## Office européen des brevets

(19)

(11) Veröffentlichungsnummer: **0 088 929**
**B1**

(12) # EUROPÄISCHE PATENTSCHRIFT

(45) Veröffentlichungstag der Patentschrift:
**02.01.85**

(21) Anmeldenummer: **83101926.0**

(22) Anmeldetag: **28.02.83**

(51) Int. Cl.³: **C 07 C 21/20, C 07 C 17/34**

(54) **Verfahren zur Herstellung von 2,3-Dichlorbutadien-(1,3).**

(30) Priorität: **11.03.82 DE 3208796**

(43) Veröffentlichungstag der Anmeldung:
**21.09.83 Patentblatt 83/38**

(45) Bekanntmachung des Hinweises auf die Patenterteilung:
**02.01.85 Patentblatt 85/1**

(84) Benannte Vertragsstaaten:
**DE FR GB IT**

(56) Entgegenhaltungen:
**DE - A - 1 618 790**
**DE - A - 2 545 341**

(73) Patentinhaber: **BAYER AG, Konzernverwaltung RP Patentabteilung, D-5090 Leverkusen 1 Bayerwerk (DE)**

(72) Erfinder: **Heinrich, Josef, Dr., Berghausener Strasse 14, D-4018 Langenfeld (DE)**
Erfinder: **Casper, Rudolf, Dr., St. Ingberter Strasse 3, D-5090 Leverkusen (DE)**
Erfinder: **Bock, Manfred, Dr., Hahnenweg 3, D-5000 Koeln 80 (DE)**

**Beschreibung**

Die vorliegende Erfindung betrifft ein verbessertes Verfahren zur Herstellung von 2,3-Dichlorbutadien-(1,3) durch Dehydrochlorierung von 2,3,4-Trichlorbuten-1 mittels einer wäßrig-alkalischen Lösung in Gegenwart von Phasentransfer-Katalysatoren und Polymerisationsinhibitoren.

Da 2,3,4-Trichlorbuten-1 mit wäßrigen Alkalien ein Zweiphasensystem bildet, verläuft die Dehydrochlorierung selbst bei starkem Rühren sehr langsam, weil die Reaktion nur an der Phasengrenzfläche stattfinden kann. Eine Steigerung der Reaktionsgeschwindigkeit läßt sich erreichen, wenn das Trichlorbuten und der Chlorwasserstoffacceptor in eine homogene Phase übergeführt werden. Derartige Verfahren sind bekannt, Literaturhinweise befinden sich in DE-OS 2 545 341.

Alle bekannten Verfahren weisen jedoch einige schwerwiegende Nachteile auf.

Das Erreichen des vollständigen Umsatzes, die Abtrennung des Dichlorbutadiens und die Erzielung einer hohen Ausbeute sind schwierig. Die Bildung von Polymerausscheidungen behindert in besonderem Maße die Abtrennung des Monomeren und verringert die Ausbeute. In der britischen Patentschrift 1 048 510 wird ausgeführt, daß die bekannten Stabilisatoren nicht in der Lage sind, die spontane, unerwünschte Polymerisation des Dichlorbutadiens bei dessen Herstellung völlig zu unterbinden.

Einen wesentlichen Fortschritt bei katalytischen Dehydrochlorierungsreaktionen brachten die Phasentransfer-Katalysatoren gemäß DE-AS 1 618 790. Als »Phasentransfer-Katalyse« werden durch Ammonium-, Phosphonium- oder Sulfoniumsalze beschleunigte Reaktionen zwischen Stoffen in einem Zweiphasensystem bezeichnet.

Der Versuch 2,3-Dichlorbutadien-(1,3) nach der in der deutschen Patentschrift 1 618 790 beschriebenen Verfahrensweise, deren wesentliche Punkte das Arbeiten unter Stickstoffatmosphäre, die Verwendung eines einzelnen Inhibitors und das Zugeben des Trichlorbutens als letzten Reaktionspartner zum Gemisch der übrigen Mischungsbestandteile sind, herzustellen, führt allerdings nicht zum angestrebten Erfolg: Im Verlaufe der Umsetzung bilden sich auch in Anwesenheit des sehr wirksamen Inhibitors Phenothiazin erhebliche Mengen an Polymerausscheidungen.

Ursache dieser starken Polymerbildung ist die bekannte extreme Polymerisationsfreudigkeit des 2,3-Dichlorbutadiens-(1,3). Die Polymerisationsgeschwindigkeit von 2,3-Dichlorbutadien-(1,3) übertrifft die des Isoprens um das 2000fache. Daher stellt die Stabilisierung dieses Monomeren gegen eine spontane, unerwünschte Polymerisation bei den während seiner Herstellung gegebenen Bedingungen ein schwer zu beherrschendes Problem dar.

Eine Lösung dieses Problems wird in der DE-OS 2 545 341 beschrieben: Dort wird ein bestimmter Reaktionstemperaturbereich (— 10 bis +60°C) eingehalten und als letzte Reaktionskomponente wird eine wäßrige Lösung des Phasentransfer-Katalysators zugegeben, wobei hochkonzentrierte Lösungen des Katalysators nicht eingesetzt werden sollen. Schließlich wird in Anwesenheit einer Inhibitorenkombination und von Luftsauerstoff gearbeitet.

Zwar gelingt es auf diese Weise, die spontane Polymerisation des 2,3-Dichlorbutadiens-(1,3) zu unterbinden, jedoch sind die nach erfolgter Zugabe des Phasentransferkatalysators erforderlichen Nachrührzeiten, die ausweislich der Beispiele 7 bis 36 Stunden betragen, aus ökonomischen Gründen nachteilig. Außerdem ist es aus Betriebssicherheitsgründen bedenklich eine Reaktion mit organischen Verbindungen in Gegenwart von Luft (etwa 20 Vol.-% Sauerstoff) durchzuführen.

Es wurde nun gefunden, daß sich die Nachrührzeiten überraschenderweise drastisch verringern lassen, wenn man die Verfahrensweise der DE-OS 2 545 341 in der Form modifiziert, daß man als letzte Reaktionskomponente die wäßrige Lösung des Alkalihydroxids zum Gemisch der übrigen Reaktionspartner hinzugibt.

Ferner wurde gefunden, daß man durch diese Variation die erforderliche Sauerstoffkonzentration in der Atmosphäre über dem Reaktionsgemisch zur Vermeidung der Spontanpolymerisation als 2,3-Dichlorbutadiens-(1,3) auf Werte unterhalb von 8 Vol.-% begrenzen kann und damit die Betriebssicherheit gewährleistet, weil bekanntermaßen für die meisten organischen Verbindungen 8 Vol.-% Sauerstoff in der Gasphase die Grenze darstellt, unterhalb der eine Explosion ausgeschlossen ist.

Gegenstand der Erfindung ist daher ein Verfahren zur Herstellung von 2,3-Dichlorbutadien-(1,3) durch Dehydrochlorierung von 2,3,4-Trichlorbuten-1 mit einer wäßrigen Lösung eines Alkalihydroxids in Anwesenheit wenigstens eines Phasentransfer-Katalysators, wenigstens eines Inhibitors und von Sauerstoff, dadurch gekennzeichnet, daß man zum Gemisch des 2,3,4-Trichlorbutens-1, des Phasentransfer-Katalysators, des Inhibitors und gegebenenfalls von Wasser die wäßrige Lösung eines Alkalihydroxids zugibt und die Reaktion bei —10 bis +60°C und unter einem Inertgas, das 0,1 bis 8 Vol.-% Sauerstoff enthält, durchführt.

Literaturhinweise auf die als Katalysator geeigneten bekannten Verbindungen sind der DE-OS 2 545 341 zu entnehmen. Von den in Frage kommenden Sulfonium-, Phosphonium- und Ammoniumverbindungen werden die Phosphonium- und Ammoniumverbindungen bevorzugt. Es handelt sich dabei um peralkylierte Phosphonium- und Ammoniumhydroxide oder -halogenide mit einem langkettigen Alkylsubstituenten, wie Tributyl-hexadecyl-ammonium-bromid.

Die zur Dehydrochlorierung benötigte Katalysatormenge hängt in starkem Maße von der Aktivität des Katalysators ab. Bei sehr aktiven Katalysatoren genügen bereits Mengen von 0,05 Gew.-%, bei weniger aktiven können Mengen bis zu 10 Gew.-%, bezogen auf 2,3,4-Trichlorbuten, eingesetzt werden.

Die Dehydrochlorierung wird vorzugsweise bei Temperaturen von −5 bis +25°C durchgeführt. Die Sauerstoffkonzentration in der Inertgasphase über dem Reaktionsgemisch liegt vorzugsweise zwischen 0,2 und 2 Vol.-%. Als Inertgas wird vorzugsweise Stickstoff eingesetzt.

Das Arbeiten mit einer Sauerstoffkonzentration, die kleiner als 0,1 Vol.-% ist, führt zu zunehmender Polymerbildung, durch die die Aufarbeitung erschwert und die Ausbeute vermindert wird.

Die bei der Durchführung des erfindungsgemäßen Verfahrens im Reaktionsgemisch vorhandene Wassermenge ist nicht kritisch. Als untere Grenze ist die Wassermenge anzusehen, die zum Lösen des entstehenden Alkalihalogenids erforderlich ist. Die obere Grenze wird bestimmt durch die Verdünnung des eingesetzten Alkalihydroxids.

Die Konzentration der eingesetzten Alkalihydroxidlösung liegt vorzugsweise zwischen 20 und 50 Gew.-%. Das Molverhältnis von 2,3,4-Trichlorbuten-1 zu Alkalihydroxyd beträgt 1 : 1,05 bis 1 : 1,5.

Als Alkalihydroxid wird bevorzugt Natriumhydroxid eingesetzt.

Eine besonders vorteilhafte Variante des erfindungsgemäßen Verfahrens besteht darin, mit den übrigen Reaktionsteilnehmern Wasser vorzulegen und Natronlauge in Form der großtechnisch zur Verfügung stehenden 50 gew.-%igen wäßrigen Natronlauge zuzugeben, so daß die effektive Natronlaugenkonzentration durch die Verdünnung im Reaktionsmedium stets klein ist. Damit wird ein sehr gleichmäßiger Reaktionsablauf erreicht, ohne daß es zu einer Verlängerung der Reaktionszeit kommt.

Vorzugsweise werden dabei 15 bis 100 Gew.-% Wasser, bezogen auf Trichlorbuten vorgelegt.

Das im Laufe der Umsetzung entstehende 2,3-Dichlorbutadien-(1,3) wird mit Polymerisationsinhibitoren stabilisiert. Geeignete Inhibitoren entstammen der Klasse der Amine, wie Phenothiazin oder Piperazin, der Klasse der Hydroxylamine, wie Diethylhydroxylamin oder der Klasse der N-Nitrosoverbindungen, wie N-Nitrosodiphenylamin.

Die Inhibitoren werden dabei in Mengen von 50 bis 10 000 ppm, vorzugsweise 100 bis 1000 ppm, bezogen auf 2,3,4-Trichlorbuten-1 eingesetzt.

Die Dehydrochlorierung wird bis zu einem Umsatz von mehr als 99,9% der Ausgangsverbindung durchgeführt, so daß weniger als 0,1 Gew.-% 2,3,4-Trichlorbuten-1 im entstandenen 2,3-Dichlorbutadien-(1,3) enthalten sind. Je nach diskontinuierlicher oder kontinuierlicher Durchführung des erfindungsgemäßen Verfahrens wird die angestrebte fast 100%ige Umsetzung der Ausgangsverbindung durch entsprechende Wahl der Reaktionsdauer bzw. der Verweilzeit erreicht.

Die Abtrennung des 2,3-Dichlorbutadiens-(1,3) erfolgt durch Phasentrennung in einem Abscheidegefäß. Bei einem kontinuierlichen Verfahren wird das Dichlorbutadien vorzugsweise in einer liegenden Trennflasche abgeschieden. Für eine schnelle Phasentrennung ist es besonders vorteilhaft, die Reaktionsmischung vor der Trennung durch ein Glaswollefilter zu schicken. Daneben können auch andere, bekannte Trennverfahren, wie Extraktion oder Destillation angewendet werden.

2,3-Dichlorbutadien-(1,3) dient zur Herstellung von Polymeren, die als Klebstoffe, speziell in der Gummi- und Metallklebung, verwendet werden. Als Comonomeres wird es bei der Herstellung kristallisationsgestörter Polychloroprentypen eingesetzt, wobei Kautschuke erhalten werden, die auch bei tiefen Temperaturen noch gute elastische Eigenschaften besitzen.

Die in den nachfolgenden Beispielen verwendete Apparatur bestand aus einem ummantelten zylindrischen 6 l Glaskolben, der mit einem Gitterrührer, Tropftrichter, Thermometer und Gaseinlaß versehen war. Am Boden des Gefäßes war ein Hahn zum Ablassen der Flüssigkeiten angebracht.

Die in der Apparatur vorhandene Luft wurde durch Stickstoff verdrängt, anschließend wurde ein Gemisch aus 0,5 Vol.-% Sauerstoff und 99,5% Vol.-% Stickstoff über das Reaktionsgemisch geleitet.

Der Umsatz des 2,3,4-Trichlorbuten-1 (TCB) wurde gaschromatographisch verfolgt.

Als Phasentransferkatalysator wurde (Bis-(2-hydroxy-propyl)-benzyl-hexadecylammoniumchlorid eingesetzt.

## Beispiel 1

1840 g TCB, 1,5 g Katalysator, 0,8 g Phenothiazin und 0,1 g N-Nitrosodiphenylamin wurden vorgelegt. Nach dem Abkühlen auf 12°C wurden unter heftigem Rühren 566 g NaOH, gelöst in 2264 g Wasser, so zugetropft, daß die Temperatur nicht über 14°C stieg. Dann wurde 3 Stunden nachgerührt und das 2,3-Dichlorbutadien-(1,3) (DCB), das noch 0,4 Gew.-% TCB enthielt, isoliert. Bei der Aufarbeitung fiel kein polymeres DCB an.

## Beispiel 2

Es wurde nach Beispiel 1 gearbeitet, jedoch mit 1 g Diethylhydroxylamin anstelle der Inhibitoren Phenothiazin und N-Nitrosodiphenylamin.

Der Umsatz betrug nach 3 Stunden Nachrührzeit über 99,9%. Polymere wurden nicht nachgewiesen.

## Beispiel 3

Es wurde nach Beispiel 1 gearbeitet, jedoch mit 1 g Piperazin anstelle der dort verwendeten Inhibitoren.

Nach einer Nachrührzeit von 3 Stunden lag der TCB-Umsatz über 99,9%. Polymeres DCB war nicht entstanden.

## Beispiel 4

1840 g TCB, 1,5 g Katalysator, 0,8 g Phenothiazin, 0,1 g N-Nitrosodiphenylamin und 1700 ml Wasser wurden vorgelegt.

Nach dem Abkühlen auf 12°C wurden unter heftigem Rühren 566 g NaOH, gelöst in 566 g Wasser, so zugetropft, daß die Innentemperatur nicht über 14°C stieg. Es wurde 4 Stunden nachgerührt. Das isolierte DCB enthielt 0,09 Gew.-% TCB. Bei der Aufarbeitung fiel kein polymeres DCB an.

## Beispiel 5

1840 g TCB, 1,5 g Katalysator, 0,8 g Phenothiazin, 0,1 g N-Nitrosodiphenylamin und 300 ml Wasser wurden vorgelegt. Nach dem Abkühlen auf 12°C wurden unter heftigem Rühren 566 g NaOH, gelöst in 566 g Wasser so zugetropft, daß die Temperatur nicht über 14°C stieg.

Es wurde 4 Stunden nachgerührt. Der TCB-Umsatz war über 99,9%. Polymere konnten nicht nachgewiesen werden.

Zur Aufarbeitung wurden 1400 ml Wasser zugegeben.

## Vergleichsbeispiel A

566 g NaOH, 2264 g Wasser, 1,5 g Katalysator, 0,8 g Phenothiazin und 0,1 g N-Nitrosodiphenylamin wurden vorgelegt. Nach dem Abkühlen auf 12°C wurden unter heftigem Rühren 1849 g TCB so zugetropft, daß die Innentemperatur nicht über 14°C stieg.

Nach einer Nachrührzeit von 4 Stunden wurde der Versuch abgebrochen.

Der Rest TCB-Gehalt im DCB betrug noch 10,3 Gew.-%. Eine geringe Menge an Polymeren lagerte sich an der Phasengrenze ab und erschwerte die Phasentrennung.

## Vergleichsbeispiel B

137,5 g NaOH, 1000 g Wasser, 500 g TCB, 0,5 g Phenothiazin, 0,2 g N-Nitrosodiphenylamin und 0,5 g Diethylhydroxylamin wurden vorgelegt.

Nach dem Abkühlen auf 12°C wurden 0,5 g Katalysator in einer 1%igen wäßrigen Lösung so zugegeben, daß die Temperatur nicht über 14°C stieg.

Nach einer Nachrührzeit von 12 Stunden wurde der Versuch abgebrochen. Der Restgehalt TCB im DCB betrug immer noch 0,3%. Bei der Aufarbeitung des Reaktionsgemisches störten geringe Mengen an Polymeren, die bezogen auf die theoretische DCB-Ausbeute etwa 1 Gew.-% ausmachten.

## Vergleichsbeispiel C

In der Versuchsapparatur wurden 1840 g TCB, 1,5 g Katalysator, 0,8 g Phenothiazin und 0,1 g N-Nitrosodiphenylamin vorgelegt.

Der im Reaktionsgefäß vorhandene Luftsauerstoff wurde durch Stickstoff verdrängt. Anschließend wurde dem Stickstoff 200 Vol.-ppm Sauerstoff zudosiert im Gegensatz zu den vorher genannten Beispielen.

Nach dem Abkühlen auf 12°C wurden unter heftigem Rühren 566 g NaOH, gelöst in 2264 g Wasser, so zudosiert, daß die Innentemperatur nicht über 14°C anstieg.

Nach 3stündiger Nachrührzeit betrug der Rest-TCB-Gehalt im DCB 0,09 Gew.-%.

An Polymeren wurden 10,4 g isoliert.

## Patentansprüche

1. Verfahren zur Herstellung von 2,3-Dichlorbutadien-(1,3) durch Dehydrochlorierung von 2,3,4-Trichlorbuten-1 mit einer wäßrigen Lösung eines Alkalihydroxids in Anwesenheit mindestens eines Phasentransfer-Katalysators, wenigstens eines Inhibitors und von Sauerstoff, dadurch gekennzeichnet, daß man zum Gemisch des 2,3,4-Trichlorbutens-1, des Phasentransfer-Katalysators, des Inhibitors und gegebenenfalls von Wasser die wäßrige Lösung eines Alkalihydroxids zugibt und die Reaktion bei −10 bis +60°C und unter einem Inertgas, das 0,1 bis 8 Vol.-% Sauerstoff enthält, durchführt.

2. Verfahren nach Anspruch 1, dadurch gekennzeichnet, daß man als Phasentransferkatalysatoren peralkylierte Phosphonium- oder Ammoniumhydroxide oder -halogenide einsetzt.

3. Verfahren nach Anspruch 1, dadurch gekennzeichnet, daß man den Katalysator in Mengen von 0,05 bis 10 Gew.-%, bezogen auf Trichlorbuten einsetzt.

4. Verfahren nach Anspruch 1, dadurch gekennzeichnet, daß man die Reaktion bei −5 bis +25°C durchführt.

5. Verfahren nach Anspruch 1, dadurch gekennzeichnet, daß man die Reaktion unter Stickstoff, der 0,2 bis 2 Vol.-% Sauerstoff enthält, durchführt.

6. Verfahren nach Anspruch 1, dadurch gekennzeichnet, daß man 20 bis 50 gew.-%ige Alkalihydroxidlösung einsetzt und das Molverhältnis von Trichlorbuten zu Alkalihydroxid 1 : 1,05 bis 1 : 1,5 beträgt.

7. Verfahren nach Anspruch 1, dadurch gekennzeichnet, daß der Inhibitor in einer Menge von 50 bis 10 000 ppm, vorzugsweise 100 bis 1000 ppm, bezogen auf Trichlorbuten eingesetzt wird.

8. Verfahren nach Anspruch 1, dadurch gekennzeichnet, daß man 15 bis 100 Gew.-% Wasser bezogen auf Trichlorbuten mit den übrigen Reaktionsteilnehmern vorlegt und das Alkalihydroxid als 50 gew.-%ige wäßrige Lösung zugibt.

9. Verfahren nach Anspruch 1, dadurch gekennzeichnet, daß man als Alkalihydroxid Natriumhydroxid einsetzt.

## Claims

1. A process for the production of 2,3-dichlorobutadiene-(1,3) by the dehydrochlorination of 2,3,4-trichlorobutene-1 with an aqueous solution of an alkali metal hydroxide in the presence of at least one phase transfer catalyst, at least one inhibitor and oxygen, characterised in that the aqueous solution of an alkali metal hydroxide is added to the mixture of the 2,3,4-trichlorobutene-1, the phase transfer catalyst, the inhibitor and optionally water, and the reaction is carried out at from −10 to +60°C under an inert gas which contains from 0.1 to 8% by volume of oxygen.

2. A process according to claim 1, characterised in that the phase transfer catalysts used are peralkylated phosphonium or ammonium hydroxides or halides.

3. A process according to claim 1, characterised in that the catalyst is used in quantities of from 0.05 to 10% by weight, based on trichlorobutene.

4. A process according to claim 1, characterised in that the reaction is carried out at from −5 to +25°C.

5. A process according to claim 1, characterised in that the reaction is carried out under nitrogen which contains from 0.2 to 2% by volume of oxygen.

6. A process according to claim 1, characterised in that a 20 to 50% by weight alkali metal hydroxide solution is used and the mol ratio of trichlorobutene to alkali metal hydroxide is from 1 : 1.05 to 1 : 1.5.

7. A process according to claim 1, characterised in that the inhibitor is used in a quantity of from 50 to 10 000 ppm, preferably from 100 to 1000 ppm, based on trichlorobutene.

8. A process according to claim 1, characterised in that from 15 to 100% by weight of water, based on trichlorobutene, are introduced with the other reaction components, and the alkali metal hydroxide is added as a 50% by weight aqueous solution.

9. A process according to claim 1, characterised in that the alkali metal hydroxide used is sodium hydroxide.

## Revendications

1. Procédé de production de 2,3-dichlorobutadiène-(1,3) par déshydrochloration de 2,3,4-trichlorobutène-1 avec une solution aqueuse d'un hydroxyde alcalin en présence d'au moins un catalyseur de transfert de phase, d'au moins un inhibiteur et d'oxygène, caractérisé en ce qu'on ajoute au mélange du 2,3,4-trichlorobutène-1, du catalyseur de transfert de phase, de l'inhibiteur et, le cas échéant, d'eau, la solution aqueuse d'un hydroxyde alcalin et on conduit la réaction à une température de −10 à +60°C et en atmosphère de gaz inerte qui contient 0,1 à 8% en volume d'oxygène.

2. Procédé suivant la revendication 1, caractérisé en ce qu'on utilise comme catalyseurs de transfert de phase des hydroxydes ou halogénures de phosphonium ou d'ammonium peralkylés.

3. Procédé suivant la revendication 1, caractérisé en ce qu'on utilise le catalyseur en quantités de 0,05 à 10% en poids par rapport au trichlorobutène.

4. Procédé suivant la revendication 1, caractérisé en ce qu'on conduit la réaction entre −5 et +25°C.

5. Procédé suivant la revendication 1, caractérisé en ce qu'on conduit la réaction sous une atmosphère d'azote qui contient 0,2 à 2% en volume d'oxygène.

6. Procédé suivant la revendication 1, caractérisé en ce qu'on utilise une solution d'hydroxyde alcalin à 20−50% en poids et le rapport molaire du trichlorbutène à l'hydroxyde alcalin a une valeur de 1 : 1,05 à 1 : 1,5.

7. Procédé suivant la revendication 1, caractérisé en ce que l'inhibiteur est utilisé en une quantité de 50 à 10 000 ppm, de préférence de 100 à 1000 ppm par rapport au trichlorobutène.

8. Procédé suivant la revendication 1, caractérisé en ce qu'on introduit préalablement 15 à 100% en poids d'eau par rapport au trichlorobutène avec les autres partenaires réactionnels et on ajoute l'hydroxyde alcalin sous la forme d'une solution aqueuse à 50% en poids.

9. Procédé suivant la revendication 1, caractérisé en ce qu'on utilise comme hydroxyde alcalin l'hydroxyde de sodium.